**Europäisches Patentamt**

**European Patent Office**

**Office européen des brevets**

(19)

(11) Veröffentlichungsnummer: **0 090 138**
**B1**

(12) **EUROPÄISCHE PATENTSCHRIFT**

(51) Int. Cl.⁴: **A 61 B 17/22**

(45) Veröffentlichungstag der Patentschrift:
**08.04.87**

(21) Anmeldenummer: **83100698.6**

(22) Anmeldetag: **26.01.83**

(54) Vorrichtung zur Zerkleinerung von Konkrementen in Körpern von Lebewesen.

(30) Priorität: **25.03.82 DE 3210919**

(43) Veröffentlichungstag der Anmeldung:
**05.10.83 Patentblatt 83/40**

(45) Bekanntmachung des Hinweises auf die Patenterteilung:
**08.04.87 Patentblatt 87/15**

(84) Benannte Vertragsstaaten:
**CH FR GB IT LI NL**

(56) Entgegenhaltungen:
**DE-A-3 038 445**
**DE-B-2 351 247**
**DE-B-2 921 444**

(73) Patentinhaber: **DORNIER SYSTEM GmbH, Postfach 1360, D-7990 Friedrichshafen (DE)**

(72) Erfinder: **Forssmann, Bernd, Dr., Salemweg 6, D-7990 Friedrichshafen 1 (DE)**
Erfinder: **Wess, Othmar, Dr., Hersbergweg 6, D-7997 Immenstaad (DE)**
Erfinder: **Zech, Hendrik, Ing.grad., Rauensteinstrasse 80, D-7770 Überlingen (DE)**
Erfinder: **Chaussy, Christian, Prof.Dr., Friedenstrasse 13a, D-8034 Germering (DE)**

(74) Vertreter: **Landsmann, Ralf, Dipl.- Ing., Kleeweg 3, D-7990 Friedrichshafen 1 (DE)**

EP 0 090 138 B1

LIBER, STOCKHOLM 1987

## Beschreibung

Vorrichtung zur Zerkleinerung von Konkrementen in Körpern von Lebewesen.

Die Erfindung betrifft eine Vorrichtung zum Zerkleinern von im Körper eines Lebewesens befindlichen Konkrementen mit einer Fokussierungskammer, die Teil eines Rotationsellipsoids ist und in deren einem Brennpunkt Stoßwellen durch Funkenentladung erzeugbar sind, wobei der Körper in einer mit Wasser gefüllten Wanne liegt.

Bekannt ist ein Gerät, bei dem die Fokussierungskammer mittels einer elastischen Membran verschlossen und mit einer Flüssigkeit, z.B. Wasser, gefüllt ist (DE-OS 23 51 247). Beim Zünden des Funkens verdampft ein Teil der Flüssigkeit (Kavitation). Bei normalem Leitungswasser reicht z.B. eine Zugspannung von $10^6$-$1,5.10^6$ Pa (10 - 15 bar) bei Impulsbelastung im Bereich von µ sec zur Blasenbildung. Ausserdem werden die in der Flüssigkeit gelösten Gase (in Wasser z.B. $N_2$, $O_2$, $CO_2$) als Bläschen freigesetzt (Pseudokavitation). Ein geringer Teil des Wassers wird auch elektrolytisch zerlegt. Durchgehende Stoßwellen verlieren an den Grenzflächen der Bläschen einen Teil ihrer Energie oder sie werden teilweise daran gebeugt, gestreut, defokussiert und erreichen nicht ihr Ziel. Auch können die Gasbläschen zwischen den Elektroden zu Unregelmässigkeiten der Funkenausbreitung führen. Diese Literaturstelle nennt keine Maßnahmen zur Behebung dieser Störungen.

Der Erfindung liegt die Aufgabe zugrunde, eine Vorrichtung zum Zerkleinern von Konkrementen in Körpern von Lebewesen zu schaffen, bei der die Stoßwellen möglichst verlustlos das Konkrement erreichen.

Die Aufgabe wird erfindungsgemäss von zwei Vorrichtungen gelöst, wobei die eine die im Anspruch 1 genannten Merkmale und die andere die im Anspruch 5 genannten Merkmale aufweist.

Die Erfindung bietet folgende Vorteile:
- hoher Wirkungsgrad, da kaum Verluste an Dampf- oder Gasblasen auftreten,
- Restgasblasen und Elektrodenabbrand werden durch den Flüssigkeitsaustausch aus der Fokussierungskammer entfernt,
- an den Funkenstrecken entstandene Dampf- und Gasblasen lösen sich durch den Überdruck schnell auf, dadurch kann die Entladungsfolge der Funkenstrecke erhöht werden,
- durch die sichere Trennung mit einer festen Membran können auch Flüssigkeiten verwendet werden, mit denen der Körper nicht in Berührung kommen darf.

Vorteilhaft ist die Kombination der drei Merkmale: Gasfreiheit, Überdruck und Strömung, die sich in ihrer Wirkung ergänzen. Daher müssen an keines der drei Merkmale sehr hohe Ansprüche gestellt werden. So muss weder die Flüssigkeit besonders hoch entgast sein und entgast gehalten werden noch muss sie besonders schnell umgewälzt oder unter besonders hohen Druck gesetzt werden, je nach Bedarf kann eines der Merkmale betont werden, dafür können die anderen Merkmale weniger ausgeprägt sein. Als Grenzfall ergibt sich die Verwendung von gashaltigem Leitungswasser bei extrem hohem Druck oder der Verzicht auf Überdruck bei extrem gasfreier Flüssigkeit.

Ausbildungen der Erfindung sind Gegenstände von Unteransprüchen.

Weitere Vorteile, Merkmale und Anwendungsmöglichkeiten werden anhand der folgenden Figuren erläutert:.

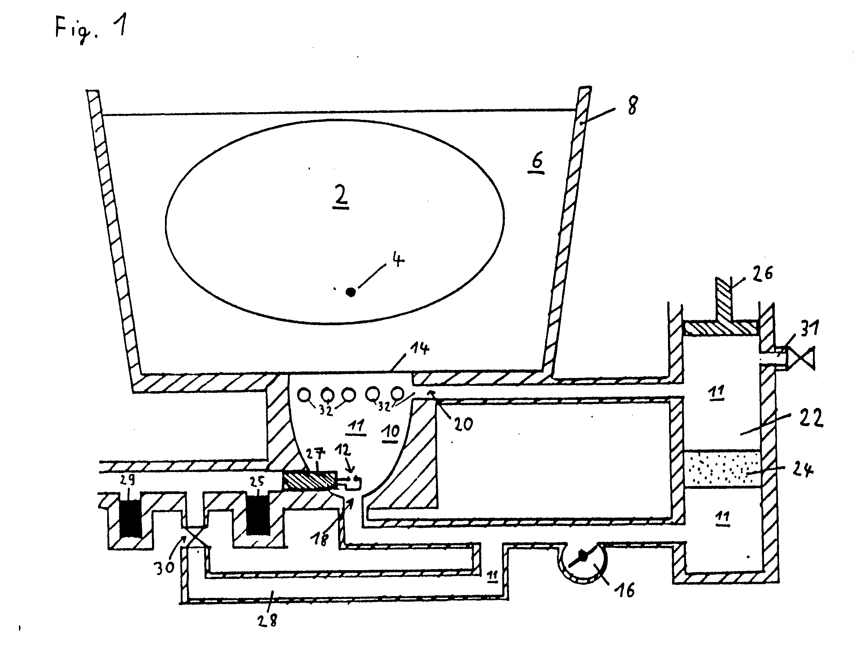

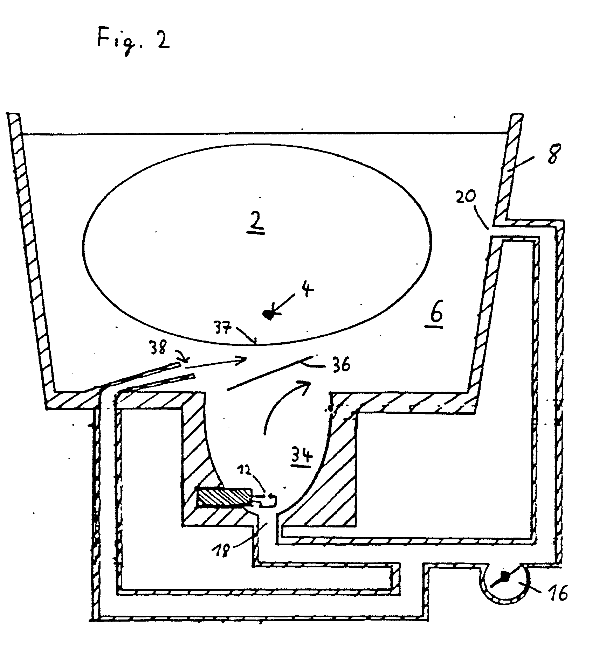

Die beiden Fig. 1 und 2 zeigen zwei Ausführungsformen der Erfindung.

Die Fig. 1 zeigt eine mögliche Ausbildung der Erfindung. Die Figur zeigt im Querschnitt einen menschlichen Körper 2, in dem sich ein Konkrement 4, z.B. ein Nierenstein, befindet. Zur verletzungsfreien Einkoppelung der Stoßwellen liegt der Körper 2 in einer mit einer Koppelflüssigkeit 6, z.B. Wasser, gefüllten Wanne 8. Gegenüber einer ellipsoidförmigen Fokussierungskammer 10, die in einem Brennpunkt eine Funkenstrecke 12 enthält, ist der Körper 2 so positioniert, dass sich das Konkrement 4 im zweiten Brennpunkt befindet. Eine Positionierungsvorrichtung ist hier nicht gezeigt. Die Fokussierungskammer 10 ist gegenüber dem Wasserbad 6 mit einer festen Membran 14 verschlossen und an einen Flüssigkeitskreislauf (Flüssigkeit 11) angeschlossen. Dieser enthält eine Umwälzpumpe 16, den Zulauf 18, den Ablauf 20, einen Vorrats- und Ausgleichsbehälter 22 mit Filter 24 und eine Vorrichtung um die Flüssigkeit unter Druck zu setzen, symbolisiert durch einen Stempel 26. Am Ausgleichsbehälter 22 ist ein Flansch 31 zum Anschluss einer Vakuumentgasungsvorrichtung (nicht gezeigt) vorgesehen. Zusätzlich kann eine Vorrichtung vorgesehen sein, die einen schnellen Austausch der Funkenstrecke 12 ohne wesentlichen Flüssigkeitsverlust erlaubt. Diese ist hier aus einem Zylinder 27, zwei Schiebern 25, 29, einer Umwegleitung 28 und einem Ventil 30 ausgebildet.

Bei der Zündung der Funkenstrecke 12 entstehen Dampf- und Gasblasen, die sich aber wegen des Überdrucks zum grössten Teil sofort wieder auflösen. Restgasblasen werden über den Flüssigkeitskreislauf von der Funkenstrecke weggespült und treten mit dem Flüssigkeitsstrom über den Abfluss 20 in den Vorratsbehälter 22. Auch Blasen, die an die Membran aufgestiegen sind und dort haften, werden vom Flüssigkeitsstrom entfernt. Im Vorratsbehälter 22 können sie sich auflösen, während an der Funkenstrecke 12 frische, blasenfreie Flüssigkeit eintritt. Besteht der Auslass 20 aus mehreren Öffnungen 32, die den gesamten Umfang der Membran umgeben, so hilft der jeder Stoßwelle folgende niederfrequente Druckstoß bei der Austreibung der Gasblasen aus der Fokussierungskammer 10.

Die stabile Abtrennung des Flüssigkeitskreislaufs in der Fokussierungskammer 10 von der Koppelflüssigkeit 6, die den Körper 2 umspült, erlaubt die Verwendung auch nichtkörperverträglicher Flüssigkeiten in der Fokussierungskammer 10. Das wichtigste Auswahlkriterium ist ein niedriger Dampfdruck der Flüssigkeit 11 der Fokussierungskammer 10. Als körperverträgliche Koppelflüssigkeit 6 kann unbehandeltes Leitungswasser verwendet werden, da die Energiedichten in der Wanne nicht so hoch sind, dass Kavitation auftritt. In grösseren Zeitabständen kann eine Entgasung der Flüssigkeit 11 durch Anschliessen einer Vakuumentgasungsvorrichtung am Flansch 31 vorgenommen werden.

Eine weitere (nicht gezeigte) Ausführung besitzt eine Fokussierungskammer mit nur einer Auslassöffnung, die in der Nähe der Membran angebracht ist. Bei dieser Ausführung ist die Fokussierungskammer gekippt angeordnet, so dass alle aufsteigenden Gasbläschen zur Öffnung im höchsten Punkt der Kammer gelangen und dort abgezogen werden. Auch hier überstreicht die Flüssigkeit die Membran und entfernt anhaftende Bläschen.

Fig. 2 zeigt eine weitere Ausführung der Erfindung bei der Verwendung von Stoßwellen schwächerer Intensität, die ohne Überdruck arbeitet. Eine abschliessende Membran ist daher nicht notwendig. Zwischen Fokussierungskammer 34 und Körper 2 ist eine schrägstehende, dünne Folie 36 befestigt. Die Fokussierungskammer 34 ist offen, Wanne 8 und Fokussierungskammer 34 werden von der gleichen Flüssigkeit 6 - hier kann z.B. leicht entgastes Wasser verwendet werden - durchspült. Als Ausgleich für den fehlenden Überdruck ist eine stärkere Umwälzung vorgesehen. Die Strömung fliesst von unten (Zufluss 18) durch die Fokussierungskammer 34, entlang der Folie 36 (Pfeil) und nimmt die dorthin aufgestiegenen Gasbläschen mit. Die Strömung aus einem zweiten Flüssigkeitsaustritt 38 (Pfeil) verhindert, dass durch Wirbelbildung an der Folie 36 Gasblasen in den Einkoppelbereich 37 strömen und entfernt Gasblasen und Verunreinigungen, die durch Manipulation des Arztes an der betreffenden Körperpartie 37 entstanden sind. Der Abfluss 20 befindet sich in der oberen Hälfte der Wanne 8. Die Strömung wird wiederum von einer Pumpe 16 aufrechterhalten.

## Patentansprüche

1. Vorrichtung zum Zerkleinern von Konkrementen in Körpern von Lebewesen mit einer Fokussierungskammer (10), die ein Teil eines Rotationsellipsoids ist und in deren einem Brennpunkt Stoßwellen durch Funkenentladung erzeugbar sind, wobei der Körper (2) in einer mit einer Flüssigkeit gefüllten Wanne liegt, dadurch gekennzeichnet, dass

a) die Fokussierungskammer (10) mit einer hochfesten Membran (14) abgeschlossen ist,

b) die Fokussierungskammer (10) mit einer weitgehend entgasten Flüssigkeit (11) gefüllt ist,

c) die Flüssigkeit (11) unter Überdruck gebracht wird,

d) die Flüssigkeit (11) umgewälzt wird, wobei die Flüssigkeit an möglichst hoher Stelle (32) der Fokussierungskammer (10) abgezogen wird und gasblasenfreie Flüssigkeit in die Fokussierungskammer nachströmt.

2. Vorrichtung nach Anspruch 1, dadurch gekennzeichnet, dass eine Vorrichtung zum Auswechseln der Funkenstrecke (12) ohne nennenswerten Flüssigkeitsverlust vorhanden ist, die z.B. aus einer Umwegleitung (28), einem Ventil (30) und Schiebern (25, 29) besteht.

3. Vorrichtung nach Anspruch 1 oder 2, dadurch gekennzeichnet, dass die Umwälzeinrichtung aus Pumpe (16), Filter (24), Ausgleichsgefäss (22), einer Einrichtung zur Druckerzeugung (26), Zulauf (18) in der Nähe der Funkenstrecke (12) und Ablauf (20) mit mehreren Öffnungen (32) in der Nähe der Membran besteht.

4. Vorrichtung nach einem der Ansprüche 1 bis 3, dadurch gekennzeichnet, dass die Fokussierungskammer (10) mit einer Flüssigkeit mit niedrigem Dampfdruck gefüllt ist.

5. Vorrichtung zum Zerkleinern von Konkrementen in Körpern von Lebewesen mit einer Fokussierungskammer (34), die ein Teil eines Rotationsellipsoids ist und in deren einem Brennpunkt Stosswellen durch Funkenentladung erzeugbar sind, wobei der Körper (2) in einer mit einer Flüssigkeit gefüllten Wanne (8) liegt, dadurch gekennzeichnet, dass die Fokussierungskammer (34) und die Wanne (8) im gleichen Flüssigkeitskreislauf liegen und die Flüssigkeit (6) an der Einkoppelstelle (37) an der die Stoßwellen in den Körper (2) eingekoppelt werden, und/oder an der Unterseite einer Folie (36), die schräg zwischen Körper (2) und Fokussierungskammer (10) angebracht ist, entlangströmt.

6. Vorrichtung nach einem der Ansprüche 1 bis 4, dadurch gekennzeichnet, dass am Ausgleichsgefäss (22) ein Anschluß (31) zur Vakuumentgasung der Flüssigkeit (11) vorgesehen ist.

## Claims

1. Apparatus for comminuting concretions in the body of a living being with a focusing chamber (10) forming part of an ellipsoid of revolution wherein shock waves are generated by spark discharge in one of the foci, the body (2) being prone in a tub filled with liquid, characterized by

a) a high-strength membrane means (14) sealing the focusing chamber (10),

b) said focusing chamber (10) being filled with a partial degassed liquid (11),

c) means for increasing the pressure on the liquid (11),

d) means for circulating the liquid (11), the liquid being evacuated at a high position (32) on the focusing chamber (10), and means for replenishing liquid free of gas bubbles into the focusing chamber.

2. Apparatus according to claim 1, including a system permitting exchanging a spark gap (12) without significant loss of liquid, said system comprising i.e. a by-pass line (28), a valve (30), and sliders (25, 29).

3. Apparatus according to claim 1 or 2, in which the circulation means comprises a pump (16), a filter (24) a compensating vessel (22), means for pressurization (26), a feed (18) in the vicinity of a spark gap (12) and a drain (20) with several openings (32) near the membrane.

4. Apparatus according to one of the claims 1 to 3, in which the focusing chamber (10) is filled with a liquid of low vapor pressure.

5. Apparatus for comminuting concretions in the body of a living being with a focusing chamber (34) forming part of an ellipsoid of revolution wherein shock waves are generated by spark discharge in one of the foci, the body (2) being prone in a tub (8) filled with liquid, characterized by a common liquid circulation means for the focusing chamber (34) and the tub (8), and inclined foil means (36) mounted between the body (2) and the focusing chamber (34), said liquid being adapted to flow along one or both of a coupling-in location (37), where the shock waves get coupled into the body (2), and the lower side of said foil means (36).

6. Apparatus according to one of the claims 1 to 4, characterized by a connection (31), for vacuum-degassing the liquid (11), at the compensating vessel (22).

**Revendications**

1. - Dispositif pour la fragmentation de concrétions à l'intérieur des corps d'organismes vivants avec une chambre de focalisation (10) faisant partie d'un ellipsoïde de révolution et dans l'un des foyers de laquelle peuvent être générées des ondes de choc par décharge à étincelles, le corps (2) étant placé dans une cuve remplie d'un liquide, caractérisé en ce que

a) la chambre de focalisation (10) est fermée par une membrane (14) très résistante,

b) la chambre de focalisation (10) est remplie d'un liquide (11) dégazé dans une large mesure,

c) le liquide (11) est mis en surpression,

d) le liquide (11) est mis en circulation, le liquide étant soutiré en un point aussi élevé que possible (32) de la chambre de focalisation (10) et du liquide exempt de bulles de gaz circulant alors dans ladite chambre de focalisation.

2. - Dispositif selon la revendication 1, caractérisé en ce qu'il comprend un dispositif pour le changement de l'éclateur (12) sans perte de liquide notable qui se compose, par exemple, d'une conduite de dérivation (28), d'une soupape (30) et de robinets-vannes (25, 29).

3. - Dispositif selon l'une des revendications 1 ou 2, caractérisé en ce que le système de circulation comprend une pompe (16), un filtre (24), un récipient de détente (22), un système de mise sous pression (26), une arrivée (18) à proximité de l'éclateur (12) et un écoulement (20) avec plusieurs orifices (32) à proximité de la membrane.

4. - Dispositif selon l'une des revendications 1 à 3, caractérisé en ce que la chambre de focalisation (10) est remplie d'un liquide à faible pression de vapeur.

5. - Dispositif pour la fragmentation de concrétions à l'intérieur des corps d'organismes vivants avec une chambre de focalisation (34) faisant partie d'un ellipsoide de révolution et dans l'un des foyers de laquelle peuvent être générées des ondes de choc par décharge à étincelles, le corps (2) étant placé dans une cuve (8) remplie d'un liquide, caractérisé en ce que la chambre de focalisation (34) et la cuvette (8) sont situées dans le même circuit de liquide et que le liquide (6) coule au point de couplage (37) où les ondes de choc sont envoyées au corps (2) et/ou le long de la face inférieure d'une feuille (36) disposée en oblique entre le corps (2) et la chambre de focalisation (34).

6. - Dispositif selon l'une des revendications 1 à 4, caractérisé en ce que le récipient de détente (22) est pourvu d'un raccordement (31) pour le dégazage sous vide du liquide (11).

Fig. 1

Fig. 2